(19) Europäisches Patentamt
European Patent Office
Office européen des brevets

(11) Veröffentlichungsnummer: **0 113 880**
**A2**

(12) **EUROPÄISCHE PATENTANMELDUNG**

(21) Anmeldenummer: 83112558.8

(22) Anmeldetag: 14.12.83

(51) Int. Cl.³: **C 07 C 103/52**, A 61 K 37/02

(30) Priorität: 17.12.82 DE 3246757

(43) Veröffentlichungstag der Anmeldung: 25.07.84 Patentblatt 84/30

(84) Benannte Vertragsstaaten: AT BE CH DE FR GB IT LI LU NL SE

(71) Anmelder: HOECHST AKTIENGESELLSCHAFT, Postfach 80 03 20, D-6230 Frankfurt am Main 80 (DE)

(72) Erfinder: Henning, Rainer, Dr., Rotenhofstrasse 31, D-6234 Hattersheim am Main (DE)
Erfinder: Urbach, Hansjörg, Dr., Le Lavandoustrasse 41, D-6242 Kronberg/Taunus (DE)
Erfinder: Becker, Reinhard, Dr., Adelheidstrasse 101, D-6200 Wiesbaden (DE)

(54) **Neue 2-Azabicyclo(2.2.1)heptan-Derivate, Verfahren zu ihrer Herstellung, diese enthaltende Mittel und deren Verwendung sowie 2-Azabicyclo(2.2.1)heptan-Derivate als Zwischenprodukte und Verfahren zu deren Herstellung.**

(57) Die Erfindung betrifft neue Derivate des 2-Aza-bicyclo[2.2.1]heptans der Formel I

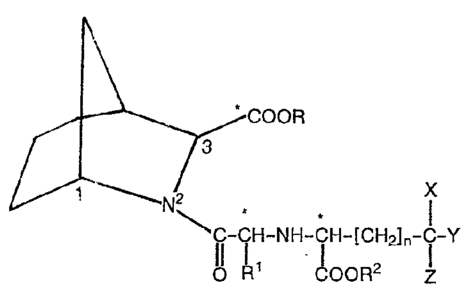

(I)

in der n = 0 oder 1, R Wasserstoff, Alkyl oder Aralkyl, $R^1$ Wasserstoff oder Alkyl, das gegebenenfalls durch Amino, Acylamino, oder Benzoylamino substituiert sein kann, Alkenyl, Cycloalkyl, Cycloalkenyl, Cycloalkyl-alkyl, Aryl oder teilhydriertes Aryl, das jeweils durch Alkyl, Alkoxy oder Halogen substituiert sein kann, Aralkyl oder Aroyl-alkyl, die beide wie vorstehend definiert im Arylrest substituiert sein können, ein mono- bzw. bicyclischer S- oder O- und/oder N-Heterocyclenrest oder eine Seitenkette einer α-Aminosäure, $R^2$ Wasserstoff, Alkenyl oder Aralkyl, Y Wasserstoff oder Hydroxy, Z Wasserstoff oder Y und Z zusammen Sauerstoff und X Alkyl, Alkenyl, Cycloalkyl, Aryl, das durch Alkyl, Alkoxy, Hydroxy, Halogen, Nitro, Amino, Alkylamino, Dialkyl-amino und/oder Methylendioxy mono-, di- oder trisubstituiert sein kann, oder 3-Indolyl bedeuten, Verfahren zu ihrer Herstellung, diese enthaltende Mittel und deren Verwendung sowie 2-Aza-bicyclo[2.2.1]heptan-Derivate als Zwischenprodukte und Verfahren zu deren Herstellung.

EP 0 113 880 A2

Neue 2-Azabicyclo[2.2.1]heptan-derivate, Verfahren zu ihrer Herstellung, diese enthaltende Mittel und deren Verwendung sowie 2-Azabicyclo[2.2.1]heptan-Derivate als Zwischenprodukte und Verfahren zu deren Herstellung

Die Erfindung betrifft neue Derivate des 2-Aza-bicyclo [2.2.1]heptans der Formel 1

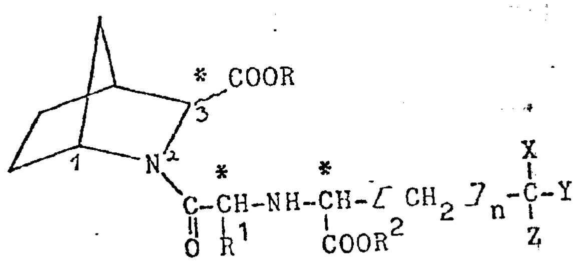

(I)

in der

n = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino, vorzugsweise ($C_1$ bis $C_6$)-Alkanoylamino oder BOC-NH, oder Benzoylamino substituiert sein kann, ($C_2$ bis $C_6$)-Alkenyl, ($C_5$ bis $C_9$)-Cycloalkyl, ($C_5$ bis $C_9$)- Cycloalkenyl, ($C_5$ bis $C_7$)-Cycloalkyl-($C_1$ bis $C_4$)-alkyl, ($C_6$-$C_{12}$)-Aryl oder teilhydriertes ($C_6$-$C_{12}$)-Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,

BAD ORIGINAL

$R^2$ = Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$-$C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino und/oder Methylendioxy mono-, di- oder trisubstituiert sein kann, oder 3-Indolyl bedeuten,

sowie deren physiologisch unbedenkliche Salze.

Als Salze kommen insbesondere Alkali- und Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Unter Aryl ist hier wie im folgenden vorzugsweise gegebenenfalls substituiertes Phenyl, Biphenylyl oder Naphthyl, insbesondere Phenyl zu verstehen; entsprechendes gilt für die Aroyl-Reste. Alkyl kann geradkettig oder verzweigt sein.

Unter einem mono- bzw. bicyclischer Heterocylen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl,Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Verbindungen der Formel I besitzen chirale C-Atome. Sowohl die R- als auch die S-Konfigurationen an allen Asymmetriezentren sind Gegenstand der Erfindung. Die Verbindungen der

Formel I können daher als optische Isomere, als Diasteromere, als Racemate oder als Gemische derselben vorliegen. Bevorzugt sind jedoch die Verbindungen der Formel I, in denen das C-Atom 3 im bicyclischen Ringsystem, sowie die mit einem Stern (*) markierten C-Atome der Seitenkette S-Konfiguration aufweisen. Im Falle (NH-CHR$^1$-CO)= Cys ist jedoch die R-Konfiguration dieses Zentrums bevorzugt.

In den bevorzugten Verbindungen kann die Carboxylgruppe in der 3-Position des bicyclischen Systems sowohl in der exoals auch in der endo-Konfiguration vorliegen. Sie weisen daher die folgenden Teilstrukturen auf:

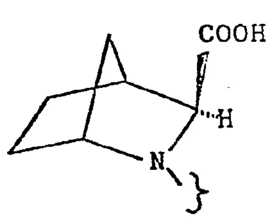

(Ia) (exo-Konfiguration)

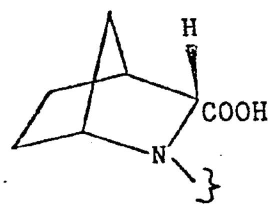

(Ib) (endo-Konfiguration)

Besonders bevorzugte Verbindungen der Formel I sind solche, in denen

n = 1

R = Wasserstoff oder Alkyl mit 1 bis 4 C-Atomen

$R_1$ = Wasserstoff, ($C_1$ bis $C_3$)-Alkyl, die gegebenenfalls acylierte Seitenkette von Lysin, ($C_2$ oder $C_3$)-Alkenyl, Benzyl, die O-($C_1$-$C_6$)-alkylierte Seitenkette von Tyrosin, 4-Methoxybenzyl, 4-Ethoxybenzyl, Phenethyl, 4-Amino-butyl oder Benzoylmethyl,

$R_2$ = Wasserstoff, ($C_1$ bis $C_4$)-Alkyl oder Benzyl und

X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)-alkyl-amino, Nitro und/oder Methylendioxy mono- oder disubstituiert oder im Falle von Methoxy, trisubstituiert sein kann, bedeuten

insbesondere solche Verbindungen der Formel I, in denen n = 1, R = Wasserstoff, $R^1$ = Methyl, 4-Methoxybenzyl oder 4-Ethoxybenzyl, $R^2$ = Wasserstoff oder Ethyl bedeuten und die chiralen C-Atome, die mit einem Stern (*) gekennzeichnet sind, die S-Konfiguration besitzen.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2 ). Im Falle, daß $R^1$ die geschützte Lysin-Seitenkette bedeutet, werden die bekannten Aminoschutzgruppen, insbesondere aber $(C_1-C_6)$-Alkanoyl bevorzugt. Im Falle, daß $R^1$ die geschützte Tyrosin-Seitenkette bedeutet, wird eine Ether-Schutzgruppe am Sauerstoff, insbesondere $(C_1-C_6)$-Alkyl, bevorzugt; besonders bevorzugte Schutzgruppen sind Methyl und Ethyl.

Die Erfindung betrifft ferner Verfahren zur Herstellung von Verbindungen der Formel I. Eine Verfahrensvariante ist dadurch gekennzeichnet, daß man eine Verbindung der Formel II,

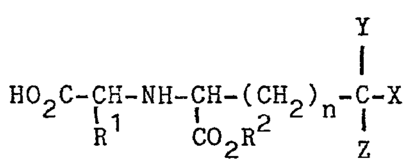

(II)

worin n, $R^1$, $R^2$, X, Y und Z die Bedeutung wie in Formel I haben mit einer Verbindung der Formel III,

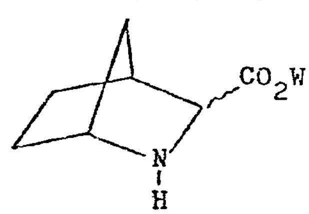

(III)

in welcher
W = Wasserstoff oder einen sauer oder hydrogenolytisch abspaltbaren Rest bedeutet, nach bekannten Amidbildungsmethoden der Peptidchemie umsetzt und gegebenenfalls anschließend durch Säurebehandlung oder Hydrierung den Rest W und gegebenenfalls durch zusätzliche Säure- oder Basenbehandlung auch den Rest $R^2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV

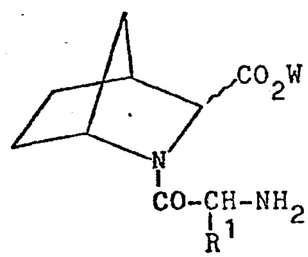

(IV)

in welcher $R^1$ die Bedeutung wie in Formel I und W die Bedeutung wie in Formel III besitzen, mit einer Verbindung der Formel V

$$R^2O_2C\text{-}CH{=}CH\text{-}CO\text{-}X \qquad (V)$$

worin $R^2$ und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest $R^2$, wie oben beschrieben, abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$$OHC\text{-}CO_2R_2 \quad (VI) \qquad X\text{-}CO\text{-}CH_3 \quad (VII)$$

worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest $R^2$ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem. Soc. 93, 2897 (1971) beschriebenen Verfahrensweise mit einer Verbindung der Formel VIII

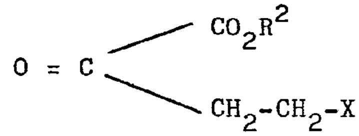

(VIII)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann elektrolytisch oder mit Reduktionsmittel wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisen erhaltenen Verbindung I mit Y und Z = zusammen Sauerstoff erhalten werden. Diese Reduktion kann mit einem Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$ bis $C_9$)-Aralkyl bedeutet, überführt werden.

Die Erfindung betrifft auch Verbindungen der Formeln IIIa und IIIb

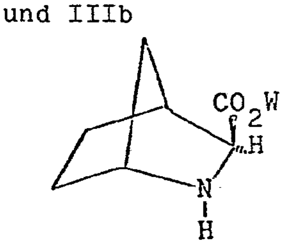

(IIIa)

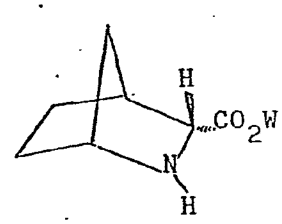

(IIIb)

worin W = Wasserstoff oder einen sauer oder hydrogenolytisch abspaltbaren Rest wie tert.-Butyl oder Benzyl bedeutet. Diese Verbindungen dienen gemäß der Erfindung als Ausgangsstoffe bei der Synthese von Verbindungen der Formel I und können erfindungsgemäß dadurch hergestellt werden, daß man

a) eine Verbindung der Formel IXa oder IXb

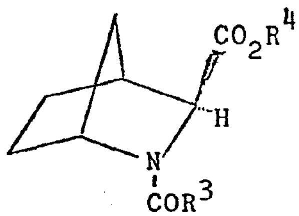

(IXa)

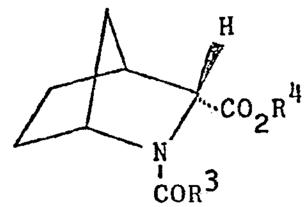

(IXb)

in welchen $R^3$ ($C_1$ bis $C_6$)-Alkyl, Phenyl oder Trifluoracetyl und $R^4$ ($C_1$ bis $C_6$)-Alkyl oder einen Benzylrest bedeuten, mit einer Mineralsäure wie Chlorwasserstoff- oder Bromwasserstoffsäure oder mit einer starken Base, wie einem Alkalihydroxid bei 20 bis 150°C, insbesondere bei 60 bis 120°C vorzugsweise in Wasser als Lösungsmittel verseift und die erhaltenen Aminosäuren IIIa bzw. IIIb (W = Wasserstoff) gegebenenfalls nach üblichen Methoden der Aminosäurechemie verestert oder ein Gemisch von Verbindungen der Formeln IXa und IXb unter den angegebenen Reaktionsbedingungen verseift und die Aminosäuren (IIIa und IIIb/W = Wasserstoff) durch an sich bekannte Methoden, wie z.B. Kristallisation oder Säulenchromatographie trennt, oder das erhaltene Aminosäurengemisch zunächst wie oben verestert und die Ester der Formeln IIIa und IIIb (W ≠ Wasserstoff) z.B. durch Chromatographie oder fraktionierte Kristallisation geeigneter Salze auftrennt oder

b) eine Verbindung der Formel X

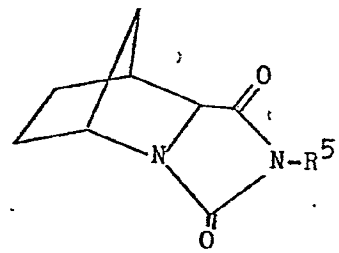

(X)

in welcher $R^5$ einen ($C_1$ bis $C_6$)-Alkyl-, ($C_6$-$C_{12}$)-Aryl-($C_1$ bis $C_3$)alkyl-, Phenyl-, 4-Methoxyphenyl- oder 4-Chlorphenylrest insbesondere einen Methyl-, Benzyl-, Phenyl- oder 4-Chlorphenylrest darstellt, mit einem Alkali- oder Erdalkalihydroxid, insbesondere Bariumhydroxid bei 20 bis 150°C, insbesondere bei 60 bis 120°C in Wasser oder Gemischen davon mit einem organischen Lösungsmittel wie Methanol, Ethanol, Isopropanol, Tetrahydrofuran oder Dioxan verseift und dann die erhaltene Aminosäure (IIIa oder b/W = Wasserstoff) gegebenenfalls nach üblichen Methoden der Aminosäurechemie verestert oder

c) eine Verbindung der Formeln XIa bzw. XIb

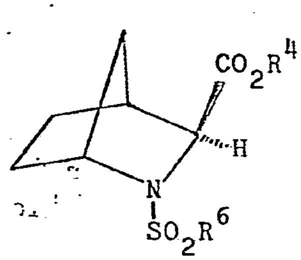

(XIa)

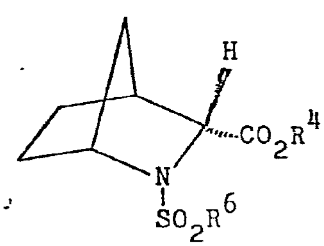

(XIb)

in welcher $R^4$ die gleiche Bedeutung wie in Formeln IXa und IXb besitzt und $R^6$ einen Phenyl-, 4-Methylphenyl- oder 4-Chlorphenylrest darstellt, zunächst mit einem Alkali-oder Erdalkalimetall, insbesondere Natrium oder Calcium in flüssigem Ammoniak bei - 60° bis -10°, dann mit

einem Alkali- oder Erdalkalihydroxid, insbesondere Natrium-, Kalium- oder Bariumhydroxid bei $20^{\circ}$ bis $120^{\circ}$ C, insbesondere 60 bis $100^{\circ}$C umsetzt, oder die Reihenfolge der beschriebenen Reaktionsschritte umkehrt und die so erhaltenen Verbindungen gegebenenfalls wie oben verestert oder

d) eine Verbindung der Formel XIIa oder XIIb

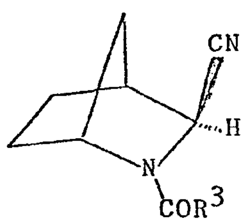

(XIIa)

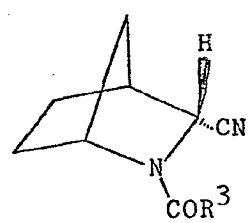

(XIIb)

mit einer Mineralsäure wie Salzsäure oder Bromwasserstoffsäure oder mit einer starken Base wie einem Alkalihydroxid bei$20^{\circ}$C bis $150^{\circ}$C, insbesondere bei 60 bis $120^{\circ}$C vorzugsweise in Wasser als Lösungsmittel verseift und die erhaltenen Aminosäuren (IIIa bzw. IIIb/W = Wasserstoff) gegebenenfalls nach üblichen Methoden der Aminosäurechemie verestert oder ein Gemisch von Verbindungen der Formeln XIIa und XIIb unter den angegebenen Reaktionsbedingungen verseift und die Aminosäuren (IIIa und IIIb/W = Wasserstoff) z.B. durch Kristallisation oder Säulenchromatographie trennt oder das erhaltene Aminosäurengemisch zunächst wie oben verestert und die erhaltenen Ester (IIIa und IIIb/W $\neq$ Wasserstoff) z.B. durch Chromatographie oder fraktionierte Kristallisation geeigneter Salze auftrennt.

Verbindungen der Formeln IXa und IXb erhält man aus den Dehydroverbindungen der Formel XIII,

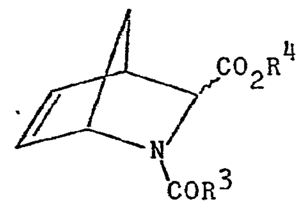

(XIII)

die aus Tetrahedron Letters 4607 (1981) bekannt sind, durch Hydrierung in einem organischen Lösungsmittel, beispielsweise Essigester in Gegenwart eines Katalysators, beispielsweise Palladium auf Tierkohle. Verbindungen der Formeln XIa und XIb erhält man auf die oben beschriebene Weise aus Verbindungen der Formel XIV,

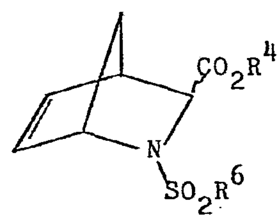

(XIV)

die aus Liebigs Ann. Chem. 1982, 960 bekannt sind. Verbindungen der Formeln XIIa und XIIb erhält man aus Verbindungen der Formel XV,

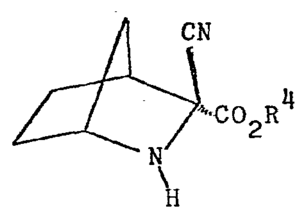

(XV)

die aus Tetrahedron Letters 3681 (1982) bekannt sind, durch Acylierung am Stickstoff mit einem Säurechlorid in Gegenwart eines Säurefängers und anschließende Decarboxyalkylierung mit einem Alkali- oder Erdalkalihalogenid und geringen Mengen Wasser in einem hochsiedenden Lösungsmittel, beispielsweise Calciumchlorid-Dihydrat in Dimethylformamid bei 160°C.

Verbindungen der Formel X erhält man analog der in Tetrahedron 27, S. 3119 (1971) beschriebenen Verfahrensweise.

Die als Ausgangsstoffe zur Herstellung der Verbindungen der Formel I verwendeten Verbindungen der Formel II mit n = 1, Y, Z = Wasserstoff, $R^1$ = Methyl und $R^2$ = Methyl oder Ethyl und X = Phenyl sind bekannt (EP-A- 37 231). Die Verbindungen der Formel II lassen sich nach verschiedenen Verfahrensweisen herstellen. Eine Synthesevariante geht von einem Keton der oben genannten Formel VII aus, das nach bekannten Verfahrensweisen in einer Mannich-Reaktion mit einer Verbindung der oben genannten Formel VI zusammen mit Aminosäureestern der Formel XVI

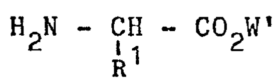

(XVI)

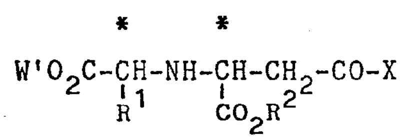

(XVII)

worin $R^1$ die oben genannte Bedeutung besitzt und W' einen hydrogenolytisch oder sauer abspaltbaren Rest, insbesondere einen Benzyl- oder einen tert.-Butyl-Rest, bedeutet, zu einer Verbindung der Formel XVII, worin $R^1$, $R^2$, X und W' die oben genannten Bedeutungen besitzen, mit der Einschränkung, daß, wenn W' einen hydrogenolytisch abspaltbaren Rest, insbesondere Benzyl, bedeutet, $R^2$ nicht die Bedeutung von W' besitzen darf, umsetzt. Spaltet man den Rest W' hydrogenolytisch mit Hilfe von beispielsweise Palladium ab, werden bei einer Wasserstoffaufnahme von 3 Moläquivalenten Verbindungen der Formel II mit X, Z = Wasserstoff erhalten. Stoppt man die Wasserstoffaufnahme bei 1 Moläquivalent, erhält man Verbindungen der Formel II mit n = 1 und Y und Z zusammen = Sauerstoff, die man ebenfalls erhält, wenn der Rest W' der Formel XVII mit Säuren, wie beispielsweise

Trifluoressigsäure oder Salzsäure, in einem inerten organischen Lösungsmittel, wie beispielsweise Dioxan, abgespalten wird.

Verbindungen der Formel XVII sind auch durch Michael-Additionen einer Verbindung der obengenannten Formel V mit einer Verbindung der obengenannten Formel XVI nach bekannten Verfahrensweisen zugänglich. Bevorzugt eignet sich dieses Verfahren zur Herstellung von solchen Verbindungen der Formel XVII, in denen $R^1$ = Methyl, $R^2$ = Ethyl und X = Aryl bedeuten.

Die Verbindungen der Formel XVII fallen als Diastereomerengemische an. Bevorzugte Diastereomere der Formel XVII sind solche, in denen die mit einem Stern markierten chiralen C-Atome jeweils S-Konfiguration aufweisen. Diese können beispielsweise durch Kristallisation oder durch Chromatographie, z.B. an Kieselgel, abgetrennt werden. Bei der nachfolgenden Abspaltung des Restes W' bleiben die Konfigurationen der chiralen C-Atome erhalten.

Die als Ausgangsstoffe zur Herstellung der Verbindungen der Formel I verwendeten Verbindungen der obengenannten Formel IV werden aus den Verbindungen der obengenannten Formel III durch Umsetzen mit einer N-geschützen 2-Aminocarbonsäure der Formel XVIII

$$V - HN - \underset{\displaystyle R^1}{\overset{}{CH}} - CO_2H \qquad \text{(XVIII)}$$

worin V eine Schutzgruppe bedeutet und $R^1$ die obengenannte Bedeutung besitzt, nach bekannten Verfahrensweisen erhalten. Als Schutzgruppe V, die nach beendeter Reaktion wieder abspalten wird, kommt beispielsweise tert.-Butoxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden sauren Abspaltung des Restes W werden als Säuren bevorzugt Trifluoressigsäure oder Chlorwasserstoff eingesetzt.

Die gemäß oben beschriebener Verfahrensweise erhaltenen Verbindungen der Formel III fallen als Gemisch an und können beispielsweise durch Umkristallisieren oder durch Chromatographie voneinander getrennt werden.

Die Verbindungen der Formel III fallen als racemische Gemische an und können als solche in die weiteren oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantionmere eingesetzt werden.

Fallen die Verbindungen der Formel I als Racemate an, können auch diese nach den üblichen Methoden wie beispielsweise über Salzbildung mit optisch aktiven Basen oder Säuren in ihre Enantiomeren gespalten oder durch Chromatographie getrennt werden.

Die erfindungsgemäßen Verbindungen der Formel I, liegen falls R = Wasserstoff ist, als innere Salze vor. Als amphotere Verbindungen können sie Salze mit Säuren oder Basen bilden. Diese Salze werden in üblicher Weise durch Umsetzen mit einem Äquivalent Säure bzw. Base hergestellt.

Die Verbindungen der Formel I und deren Salze besitzen lang andauernde, intensive blutdrucksenkende Wirkung. Sie sind

starke Hemmer des Angiotensin-Converting-Enzyms (ACE-Hemmer). Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-500 mg, vorzugsweise bei 5-80 mg, insbesondere 5 - 40 mg je Einzeldosis bei einem normalgewichtigen erwachsenen Patienten; dies entspricht ca. 13 - 6500 µg/kg/Tag, vorzugsweise 65 bis 1000 µg/kg/Tag, insbesondere 65 bis 500 µg/kg/Tag. Die Dosis kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche

Salze, gewünschtenfalls mit den dafür üblichen Substanzen wie Lösungsvermittler, Emulgatoren, oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glukose- oder Mannitlösungen, oder auch eine Mischung aus den verschiedenen genannten Lösungsmitteln.

Die außerordentlich starke Wirksamkeit der Verbindungen gemäß Formel I wird durch die pharmakologischen Daten der nachfolgenden Tabelle belegt:

Intraduodenale Gabe an der narkotisierten Ratte, 50 % Hemmung der durch 310 ng Angiotensin I ausgelösten Pressorreaktion 30 min. nach Applikation in der Dosis .............= $ED_{50}$:

Tabelle

Die aufgeführten Verbindungen besitzen die S-Konfiguration an allen mit einem Stern gekennzeichneten C-Atomen ; "Konfiguration" bezieht sich auf die Stellung der Carboxylgruppe an bicyclischen Ringsystem.

| n | X | Y | Z | $R^1$ | $R^2$ | R | Konfiguration | $ED_{50}$ (μg/kg) |
|---|---|---|---|---|---|---|---|---|
| 1 | $C_6H_5$ | H | H | $C_2H_5$ | $CH_3$ | H | exo | 700 |
| 1 | $C_6H_5$ | H | H | $C_2H_5$ | $CH_3$ | H | endo | 500 |
| 1 | $C_6H_5$ | H | H | H | $CH_3$ | H | exo | 1800 |
| 1 | $C_6H_5$ | H | H | H | $CH_3$ | H | endo | 1600 |
| 1 | $C_6H_5$ | -O- | | $C_2H_5$ | $CH_3$ | H | exo | 900 |
| 1 | $C_6H_5$ | -O- | | $C_2H_5$ | $CH_3$ | H | endo | 750 |

Die in den folgenden Beispielen angegebenen $^1H$-NMR-Daten wurden, wenn nicht anders angegeben, durch Messung in $CDCl_3$ ermittelt und sind in $\delta$ (ppm) angegeben.

Beispiel 1

2-Azabicyclo- [2.2.1]-carbonsäure

a) 3-Carbethoxy-3-cyano-2-p-toluolsulfonyloxy-2-azabicyclo [2.2.1]hept-5-en

12 g frisch gecracktes Cyclopentadien und 18 g p-Toluolsulfonyloximino-cyanessigsäureethylester werden in 120 ml Toluol gelöst und 6 Std. auf 80°C erwärmt. Das dunkel gefärbte Gemisch wird eingeengt und über Kieselgel mit Essigester/Cyclohexan (1:1) als Laufmittel chromatographiert. Man erhält 8,4 g farblose Kristalle von Schmelzpunkt 112°C.

b) 3-Carbethoxy-3-cyano-2-p-toluolsulfonyloxy-2-azabicyclo [2.2.1]heptan

4,5 g 3-Carbethoxy-3-cyano-2-p-toluolsulfonyloxy-2-azabicyclo [2.2.1] hept-5-en werden in 270 ml Essigester mit Palladium auf Tierkohle (10 %) hydriert. In 10 Minuten wird 1 Mol Wasserstoff aufgenommen. Nach Filtrieren wird eingeengt. Man erhält 4,4 g der Titelverbindung als langsam kristallisierendes Öl.

c) 3-Carbethoxy-3-cyano-2-azabicyclo [2.2.1]heptan

11,6 g 3-Carbethoxy-3-cyano-2-p-toluolsulfonyloxy-2-azabicyclo [2.2.1] heptan werden in 500 ml THF und 50 ml Wasser gelöst. Man gibt Aluminiumamalgam, das aus 8.9 g Aluminium hergestellt wurde, zu, rührt 3 Stunden bei Raumtemperatur, saugt über Klärschicht ab und engt ein. Der Rückstand wird in Dichlormethan/

Wasser aufgenommen, noch zweimal mit Dichlormethan extrahiert, über Natriumsulfat getrocknet und eingeengt. Man erhält 5,2 g der Titelverbindung als blaßgelbes Öl.

| | | |
|---|---|---|
| $^1$H-NMR Daten: | 4,2 | (q, 2H) |
| | 3,5 | (s, 1H) |
| | 2,9-2,5 | (m, 2H) |
| | 2,1-1,2 | (m, 6H) |
| | 1,3 | (t, 3H) |

d) <u>2-Acetyl-3-carbethoxy-3-cyano- 2-azabicyclo [2.2.1] heptan</u>

5,2 g 3-Carbethoxy-3-cyano-2-azabicyclo [2.2.1] heptan werden in 100 ml Tetrahydrofuran zusammen mit 3,25 g Triethylamin gelöst. Unter Eiskühlung werden 2,3 g Acetylchlorid zugetropft. Nach 4 Stunden bei 0°C wird mit 1 N Salzsäure angesäuert, mit Dichlormethan dreimal extrahiert, mit Natriumsulfat getrocknet und eingeengt. Das Rohprodukt wird an Kieselgel mit Essigester/Cyclohexan (1:1) als Laufmittel gereinigt. Man erhält 5,1 g der Titelverbindung als farbloses Öl.

$^1$H-NMR-Daten: 4,33 (br. s, 1H);
4,2 (q, 2H);
2,9 (s, 1H);
2,1 (s, 3H);
2,4-1,4 (m, 6H);
1,3 (t, 3H).

e) <u>2-Acetyl-3-cyano-2-azabicyclo [2.2.1] heptan</u>

5,1 g 2-Acetyl-3-carbethoxy-3-cyano-2-azabicyclo[2.2.1] heptan werden in 100 ml Dimethylformamid gelöst. Nach Zugabe von 15,8 g Calciumchlorid-Dihydrat wird 90

Minuten auf 170°C erhitzt. Nach Abkühlen wird mit Wasser verdünnt, mit Dichlormethan extrahiert, der Extrakt viermal mit Wasser gewaschen, über Natriumsulfat getrocknet und eingeengt. Man erhält 2,85 g der Titelverbindung als gelbes Öl.

$^{1}$H-NMR-Daten: 4,5-4,0 (m, 2H);
2,5 (s, 3H);
2,3-1,4 (m, 6H).

f) <u>2-Azabicyclo [2.2.1]heptan-3-carbonsäure</u>

2,8 g 2-Acetyl-3-cyano-2-azabicyclo [2.2.1] heptan werden in 55 ml 5 N Salzsäure 4 Stunden am Rückfluß erhitzt. Nach Einengen zur Trockne wird in Wasser aufgenommen und durch Zugabe von Ionenaustauscher (Amberlite$^{R}$IRA 93, $OH^-$-Form) auf einen pH-Wert von 5,8 gestellt. Nach Filtrieren und Einengen wird mit Ethanol/Aceton verrieben. Die Titelverbindung fällt als Gemisch des endo- und des exo-Isomeren im Verhältnis von 1:1 an, Ausbeute 2 g.

Rf-Werte (Kieselgel, Merck-Fertigplatten, Laufmittel:
Dichlormethan/Methanol/Wasser/ Eisessig = 10:5 : 1:1)
exo-Isomer : 0,41
endo-Isomer: 0,35

<u>Beispiel 2</u>

<u>2-Azabicyclo [2.2.1]heptan-3-carbonsäurebenzylester</u>

2 g 2-Azabicyclo [2.2.1] heptan-3-carbonsäure (Isomerengemisch) werden zu einer bei -5°C hergestellten Lösung von 2 ml Thionylchlorid in 20 ml Benzylalkohol gegeben. Nach

Stehen über Nacht wird mit Wasser versetzt und mit Essigester fünfmal extrahiert. Die wäßrige Lösung wird mit Natriumhydrogencarbonatlösung auf einen pH-Wert von 8 gestellt, mit Dichlormethan extrahiert, über Natriumsulfat getrocknet und eingeengt. Man erhält 1,82 g Titelverbindung als farbloses Öl, das als Isomerengemisch anfällt.

$R_f$-Werte (Kieselgel, Merck-Fertigplatten, Laufmittel Dichlormethan/Methanol = 9 : 1)
exo-Isomer : 0,44
endo-Isomer: 0,36

Beispiel 3

2-Azabicyclo [2.2.1]heptan-3-exo-carbonsäurebenzylester

Erhalten durch Säulenchromatographie an Kieselgel aus dem Isomerengemisch von Beispiel 2 (Laufmittel Dichlormethan/Methanol 9:1, weniger polares Isomer.)
0,89 g farbloses Öl.

$^1$H-NMR-Daten: 7,33 (s, 5H)
5,15 (s, 2H)
3,53 (br. s, 1H)
3,36 (s, 1 H)
2,64 (br. s, 1H)
2,13 (s, 1H)
1,9-1,1 (m, 6H)

Beispiel 4

2-Azabicyclo [2.2.1]heptan-3-endo-carbonsäurebenzylester

Erhalten wie bei Beispiel 3 beschrieben aus den Isomerengemisch von Beispiel 2; 0,84 g farbloses Öl

$^1$H-NMR-Daten: 7,36 (s, 5H)
5,20 (s, 2H)
3,90 (d, J = 4 Hz, 1H)
3,51 (br.s, 1H)
2,67 (br. s, 1H)
2,30 (s, 1H)
2,11-1,1 (s, 6H).

Beispiel 5

N-(1-S-Carbethoxy-3-phenyl-propyl)-S-alanyl-2-azabicyclo [2.2.1] heptan-3-exo-carbonsäurebenzylester (Diastereomer A 5) und N-(1-S-Carbethoxy-3-phenyl-propyl)-S-alanyl-2-azabicyclo [2.2.1] heptan-3-exo-carbonsäurebenzylester (Diastereomer B 5)

1,14 g N-(1-S-Carbethoxy-3-phenyl-propyl)-S-alanin, 0,48 g N-Hydroxybenzotriazol, 0,89 g 2-Azabicyclo [2.2.1] heptan-3-exo-carbonsäurebenzylester sowie 0,89 g Dicyclohexylcarbodiimid werden bei Raumtemperatur in 17 ml Dimethylformamid gelöst und 3 Stunden gerührt. Nach Verdünnen mit 20 ml Essigester wird je einmal mit 10%iger Zitronensäure, 1 N Natriumhydrogencarbonat und gesättigter Kochsalzlösung gewaschen, über Natriumsulfat getrocknet und eingeengt. Man erhält 1,62 g eines Isomerengemisches, das durch Chromatographie an Kieselgel mit Essigester/Cyclohexan (1:1) als Laufmittel getrennt wird.

Diastereomer A5: 0,6 g , Rf-Wert: 0,24
$^1$H-NMR-Daten: 7,10 (s, 5H)
7,00 (s, 5H)
5,00 (s, 2H)
4,5-3,9 (m, 3H)
3,8-3,1 (m, 2H)
3,0-1,3 (m, 12H)
1,32 (d+t, J.7Hz, 6H).

Diastereomer B 5: 0,51 g, Rf-Wert = 0,17

1H-NMR-Daten: 7,10 (s, 5H)
7,00 (s, 5H)
5,00 (s, 2H)
4,5-3,9 (m, 3H)
3,8.3,0 (m, 2H)
3,0-1,2 (m, 12H)
1,4-1,1 (m, 6H)

Beispiel 6

N-(1-S-Carbethoxy-3-phenyl-propyl)-S-alanyl-2-azabicyclo [2.2.1] heptan-3-endo-carbonsäurebenzylester (Diastereomer A 6) und N-(1-S-Carbethoxy-3-phenyl-propyl)-S-alanyl-2-azabicyclo [2.2.1] heptan-3-endo-carbonsäurebenzylester (Diastereomer B 6)

1,05 g N-(1S-Carbethoxy-3-phenyl-propyl)-S-alanin, 0,46 g N-Hydroxybenztriazol, 0,82 g 2-Azabicyclo [2.2.1] heptan-3-endocarbonsäurebenzylester und 0,82 g Dicyclohexylcarbodiimid werden in 11 ml Dimethylformamid analog dem in Beispiel 5 beschriebenen Verfahren umgesetzt. Säulenchromatographie an Kieselgel (Laufmittel Essigester/Cyclohexan 1:1) liefert:

Diastereomer A 6: 0,46 g, Rf-wert: 0,21

1H-NMR-Daten: 7,10 (s, 5H);
7,00 (s, 5H);
5,05 (s, 2H);
4,4-3,8 (m, 3H);
3,8-3,9 (m, 2H);
2,8-1,3 (m, 12H);
1,4-1,1 (m, 6H).

Diastereomer B 6 : 0,46 g, Rf-Wert 0,10

$^1$H-NMR-Daten 7,15 (s, 5 H)
7,00 (s, 5 H)
5,8 (s, 2 H)
4,4-3,8 (m, 3 H)
3,8-3,1 (m, 2 H)
3,0-1,3 (m, 12 H)
1,3-1,0 (m, 6 H)

Beispiel 7

N-(1-S-Carbethoxy-3-phenyl-propyl)-S-alanyl-2-azabicyclo [2.2.1] heptan-3-S-exo-carbonsäure Hydrochlorid

0,6 g des Diastereomers A 5 aus Beispiel 5 werden in 50 ml Ethanol mit 250 mg Palladium auf Tierkohle bei Raumtemperatur und Normaldruck 1 Stunde hydriert. Nach Abfiltrieren wird mit 2,5 N ethanolischer Salzsäure auf pH 2 gestellt und eingeengt. Der Rückstand wird in wenig Dichlormethan aufgenommen und mit Diisopropylether gefällt, 0,6 g farbloses Pulver, F. 105 - 106°C (Zers.)

$^1$H-NMR-Daten (DMSO-$c_6$): 7,26 (s, 6 H)
4,5 (br. s, 1 H)
4,25 (q, J = 7Hz, 2 H)
3,9-3,0 (m, 2 H)
3,0-1,3 (m, 12 H)
1,4 (d, J = 6Hz, 3 H)
1,26 (t, J = 7Hz, 3 H)

Massenspektrum (trimethylsilylierte Verbindung):
m/e = 474 ($m^+$), 459 ($M^+-CH_3$), 401, 329, 269, 234 (100 %), 160, 147, 117, 96, 91, 56.

Beispiel 8

N-(1-S-Carbethoxy-3-phenyl-propyl)-S-alanyl-2-azabicyclo [2.2.1] heptan-3-R-exo-carbonsäure-Hydrochlorid

0,51 g des Diastereomeres B 5 aus Beispiel 5 werden analog dem in Beispiel 7 beschriebenen Verfahren hydriert.
0,38 g farbloses Pulver, F. 105 - 106°C (Zers.)

$^1$H-NMR-Daten (DMSO-$d_6$): 7,25 (s, 5 H)
4,6-4,3 (m, 1 H)
4,25 (q, 2 H)
4,0-3,0 (m, 3 H)
3,5-1,5 (m, 12 H)
1,5-1,1 (d+t, 6 H).

Massenspektrum (trimethylsilylierte Verbindung)
m/e: 474 ($M^+$), 459 ($M^+$-$CH_3$), 401, 384, 280, 234 (100 %), 160, 117, 96, 91, 56

Beispiel 9

N-(1-S-Carbethoxy-3-phenyl-propyl)-S-alanyl-2-azabicyclo [2.2.1] heptan-3-S-endo-carbonsäure-Hydrochlorid

0,46 g des Diastereomers A 6 aus Beispiel 6 werden analog dem im Beispiel 7 beschriebenen Verfahren hydriert.
0,35 g farbloses Pulver
$^1$H-NMR-Daten (DMSO-$d_6$): 7,25 (s, 5 H)
4,6-3,2 (m, 5 H)
3,0-1,3 (m, 12 H)
1,4 (d, J = 6 Hz, 3 H)
1,25 (t, J = 6 Hz, 3 H)

Massenspektrum (trimethylsilylierte Verbindung)
m/e: 474 ($M^+$), 459 ($M^+$-$CH_3$), 401, 369, 357, 329, 269, 234 (100 %), 160, 117, 96, 91

Beispiel 10

N-(1-S-Carbethoxy-3-phenyl-propyl)-S-alanyl-2-azabicyclo [2.2.1] heptan-3-R-endo-carbonsäure-Hydrochlorid

0,46 g des Diastereomers B 6 aus Beispiel 6 werden analog den in Beispiel 7 beschriebenen Verfahren hydriert. 0,32 g farbloses Pulver.

$^1$H-NMR-Daten (DMSO-$d_6$): 7,2 (s, 5 H)
4,6-3,2 (m, 5 H)
3,0-1,2 (m, 12 H)
1,6-1,1 (d+t, 6 H)

Massenspektrum (trimethylsilylierte Verbindung)
m/e: 474 ($M^+$), 459 ($M^+$-$CH_3$), 401, 384, 329, 280, 234 (100 %), 160, 117, 91

Beispiel 11

2-Azabicyclo[2.2.1]heptan-3-exo-carbonsäure-tert. butylester Hydrochlorid

Zu einer auf -10°C gekühlten Lösung von 1,1 g 2-Azabicyclo [2.2.1] heptan-3-carbonsäure in 10 ml Dioxan werden 1 ml konz. Schwefelsäure und 5 g Isobuten gegeben. Das Reaktionsgemisch wird im Autoklaven langsam auf 25°C erwärmt und 20 Stunden bei dieser Temperatur gehalten. Man gibt das Gemisch in eiskaltes 50%iges wäßriges Natriumhydroxid und extrahiert mit Dichlormethan. Nach Waschen der organischen Phase mit Wasser wird getrocknet, eingeengt, der Rückstand in Ether gelöst und gasförmiger Chlorwasserstoff eingeleitet. Das Produkt (Isomerengemisch aus

exo und endo) wird abgesaugt. Man erhält 0,74 g der Titelverbindung.
$^1$H-NMR-Daten(DMSO-$d_6$): 3,9-3,6 (m, 1 H)
3,9-2,6 (m, 1 H)
2,2-1,3 (m, 7 H)
1,3 (s, 9 H).

Beispiel 12

2-Azabicyclo [2.2.1]heptan-3-endo-carbonsäure-tert. butylester Hydrochlorid

Analog der in Beispiel 11 beschriebenen Verfahrensweise werden aus 1,8 g 2-Azabicyclo [2.2.1] heptan-3-endo-carbonsäure 1,4 g der Titelverbindung erhalten.
$^1$H-NMR-Daten (DMSO-$d_6$): 3,8-3,5 (m, 1 H)
2,9-2,5 (m, 1 H)
2,3-1,3 (m, 7 H)
1,3 (s, 9H).

Beispiel 13

N-(1-S-Carbethoxy-3-phenyl-propyl)-S-alanyl-2-azabicyclo [2.2.1] heptan-3S-exo-carbonsäure-tert. butylester (Diastereomere A 13)

Analog der Verfahrensweise von Beispiel 5 erhält man aus 0,5 g 2-Azabicyclo [2.2.1] heptan-3-exo-carbonsäure-tert. butylester. Hydrochlorid, 0,54 g N-(1-S-Carbethoxy-3-phenylpropyl)-S-alanin, 0,23 g N-Hydroxybenzotriazol sowie 0,40 g Dicyclohexylcarbodiimid unter Zusatz von 0,20 g N-Ethylmorpholin 0,26 g der Titelverbindung als farbloses Öl.

$^1H$-NMR-Daten: 7,1 (s, 5 H);
4,5-4,1 (m, 1 H);
4,1 (q, J=7Hz, 2 H);
3,9-1,9 (m, 14 H);
1,3 (s, 9 H);
1,2 (d+t, J=7Hz, 6 H).

Beispiel 14

N-(1-S-Carbethoxy-3-phenyl-propyl)-S-alanyl-2-azabicyclo [2.2.1] heptan-3-exo-carbonsäure-Hydrochlorid

0,26 g des tert. Butylesters aus Beispiel 5 werden in 1,5 ml Trifluoressigsäure gelöst und bei 0°C 3 Stunden gerührt. Die Trifluoressigsäure wird im Vakuum abgedampft, Toluol zugesetzt und nochmals eingeengt. Nach Filtration über eine kurze Kieselgelsäule mit Methylenchlorid/Ethanol (10:1) als Laufmittel wird mit ethanolischer Salzsäure sauer gestellt und eingeengt. Nach Lösen in wenig Methylenchlorid wird die Titelverbindung mit Diisopropylether ausgefällt. Man erhält 0,21 g, identisch mit der Verbindung aus Beispiel 7.

Beispiel 15

N-(1-S-Carboxy-3-phenyl-propyl)-S-alanyl-2-azabicyclo [2.2.1] heptan-3-S-exo-carbonsäure

Eine Lösung von 0,2 g N-(1-S-Carbethoxy-3-phenyl-propyl) -S-alanyl-2-azabicyclo [2.2.1] heptan-3-S-exo-carbonsäure · Hydrochlorid in 2 ml Wasser wird mit zwei Äquivalenten Kaliumhydroxid und einem 10 %igen Überschuß 4n Kaliumhydroxidlösung versetzt. Nach 8-stündigem Rühren bei 20 bis 25°C wird die Reaktionslösuing mit 2n Salzsäure auf einen pH-Wert von 4 gestellt und im Vakuum eingeengt. Man nimmt den Rückstand in Essigester auf und filtriert das abgeschiedene Salz ab. Die Essigesterlösung wird eingeengt, der Rückstand mit Diisopropylether verrieben und abgesaugt.

$^1$H-NMR-Daten: 7,1 (s, 5 H);
4,4-4,0 (m, 1 H);
3,9-1,3 (m, 14 H);
1,2 (d, 3 H).

Beispiel 16

N-(1-S-Carboxy-3-phenyl-propyl)-S-alanyl-2-azabicyclo [2.2.1] heptan-3-S-endo-carbonsäure

Eine Lösung von 0,16 g N-(1-S-Carbethoxy-3-phenyl-propyl)-S-alanyl-2-azabicyclo [2.2.1] heptan-3S-endo-carbonsäure Hydrochlorid wird in 2 ml Wasser mit 2,2 Äquivalenten Kaliumhydoxid analog der in Beispiel 15 beschriebenen Verfahrensweise umgesetzt. Man erhält 0,11 g der Titelverbindung.

$^1$H-NMR-Daten: 7,1 (s, 5 H);
4,4-4,0 (m, 1 H);
3,9-1,2 (m, 14 H);
1,25 (d, 3 H).

Beispiel 17

N-(1-S-Carbethoxy-3-phenyl-3-oxo-propyl)-S-alanyl-2-azabicyclo [2.2.1] heptan-3-S-endo-carbonsäure-tert. butylester

0,24 g 2-Azabicyclo [2.2.1] heptan-3-endo-carbonsäure-tert. butylester Hydrochlorid werden zusammen mit 0,14 g

1-Hydroxybenzotriazol, 0,29 g N-(1-S-Carbethoxy-3-phenyl-3-oxo-propyl)-S-alanin, 0,22 g Dicyclohexylcarbodiimid und 0,12 g N-Ethylmorpholin in 3 ml DMF gelöst und 3 Stunden bei 20°C gerührt. Nach Verdünnen mit Essigester wird filtriert, zweimal mit Wasser gewaschen, getrocknet und eingeengt. Der Rückstand wird an Kieselgel mit Cyclohexan/Essigester (1:1) als Laufmittel chromatographiert; man erhält 0,16 g der Titelverbindung.

$^1$H-NMR-Daten: 8,2-7,1 (m, 5 H);
4,7-4,1 (m, 1 H);
4,1 (q, J=7Hz, 2 H)
3,9-1,3 (m, 12 H);
1,3 (s, 9 H);
1,2 (d+t, 6 H).

## Beispiel 18

N-(1-S-Carbethoxy-3-phenyl-3-oxo-propyl)-S-alanyl-2-azabicyclo [2.2.1] heptan-3-S-endo-carbonsäure. Hydrochlorid

0,16 g der Verbindung aus Beispiel 17 werden analog dem Verfahren von Beispiel 14 mit 2 ml Trifluoressigsäure zu 0,12 g der Titelverbindung umgesetzt.

$^1$H-NMR-Daten in DMSO-$d_6$: 8,1-7,2 (m, 5 H);
4,7-4,1 (m, 1 H);
4,1 (q, J=7Hz, 2 H)
3,9-3,1 (m, 12 H);
1,25 (d+t, 6 H).

Beispiel 19

N-(1-S-Carboxy-3-phenyl-3-oxo-propyl)-S-alanyl-2-azabicyclo [2.2.1] heptan-3-S-endo-carbonsäure

0,19 g der Verbindung aus Beispiel 9 werden mit 2,2 Äquivalenten Kaliumhydroxid analog der in Beispiel 7 beschriebenen Verfahrensweise umgesetzt.

$^1$H-NMR-Daten: 8,1-7,2 (m, 5 H);
4,7-4,1 (m, 1 H);
3,9-3,1 (m, 12 H);
1,2 (d, J=7Hz, 3 H).

Beispiel 20

S-Alanyl-2-azabicyclo [2.2.1] heptan-3-S-exo-carbonsäure-tert.butylester

a) N-Methylsulfonylethyloxycarbonyl (MSC)-S-alanyl-2-azabicyclo [2.2.1] heptan-3-S-exo-carbonsäure-tert. butylester

Zu einer Lösung von 10 g MSC-Ala-OH in 50 ml Dimethylformamid werden 6,7 g 1-Hydroxybenzotriazol und 14,0 g 2-Azabicyclo [2.2.1] heptan-3-exo-carbonsäure-tert. butylester gegeben. Der pH-Wert wird mit N-Ethylmorpholin auf 8,0 eingestellt. Das Gemisch wird im Eisbad gekühlt und mit 10,5 g Dicyclohexylcarbodiimid versetzt. Man rührt 15 Stunden bei 20-25°C. Der ausgefallene Harnstoff wird abgesaugt, das Filtrat im Vakuum eingeengt, und in Essigester aufgenommen. Die organische Phase wird mit nacheinander mit Kaliumhydrogensulfat-, Kaliumhydrogencarbonat- und Natriumchloridlösung gewaschen, getrocknet und eingedampft. Der Rückstand

wird an Kieselgel mit Essigester/Cyclohexan 1:1 chromatographiert.
Ausbeute: 10 g

$^1$H-NMR-Daten: 4,8-3,8 (m, 2 H);
3,8-3,1 (m, 5 H);
3,0 (s, 3 H);
2,9-1,2 (m, 7 H);
1,4 (s, 9 H);
1,2 (d, J= 7Hz, 3 H).

b) <u>S-Alanyl-2-azabicyclo [2.2.1] heptan-3-S-exo-carbonsäure-tert.butylester</u>

2.0 g der Verbindung aus Beispiel 20 a werden in 15 ml Methanol und 1,5 ml Wasser gelöst. Es wird mit 2n Natronlauge auf pH 13 gebracht und 2 Stunden bei Raumtemperatur gerührt. Danach wird mit 2n Salzsäure neutralisiert, das Methanol im Vakuum abgedampft, die wäßrige Phase mit Essigester extrahiert, die Essigesterlösung mit Wasser gewaschen, getrocknet und eingeengt. Der Rückstand wird über Kieselgel mit Essigester als Elutionsmittel filtriert.

$^1$H-NMR-Daten: 4,7-4,2 (m, 1 H);
3,9-3,3 (m, 2 H);
2,9-1,2 (m, 7 H);
1,4 (s, 9 H);
1,2 (d, J= 7Hz, 3 H).

<u>Beispiel 21</u>

<u>N-(1-S-Carbethoxy-3-oxo-3-phenyl-propyl)-S-alanyl-2-azabicyclo [2.2.1] heptan-3-S-exo-carbonsäure-tert.butylester</u>

5 m Mol der Verbindung aus Beispiel 20 b) werden zusammen

mit 5 m Mol 3-Benzoyl-acrylsäureethylester und 5 Tropfen Triethylamin in 50 ml wasserfreiem Ethanol gelöst und das Gemisch 24 Stunden bei 20 bis 25°C gerührt. Es wird zur Trockene eingedampft und der Rückstand in Essigester aufgenommen. Nun wird mit Wasser gewaschen, getrocknet und eingedampft.

Das Diastereomerengemisch wird an Kieselgel mit Essigester/Cyclohexan als Elutionsmittel chromatographiert.

$^1$H-NMR-Daten: 8,2-7,3 (m, 5 H);
4,7-4,0 (m, 1 H);
4,2 (q, J= 7Hz, 2 H);
3,9-1,3 (m, 12 H);
1,3 (s, 9 H);
1,2 (d+t, 6H).

Beispiel 22

N-(1-S-Carbethoxy-3-phenyl-propyl)-S-alanyl-2-azabicyclo-[2.2.1] heptan-3-S-exo-carbonsäure-tert.butylester

5 mmol S-Alanyl-2-azabicyclo [2.2.1] heptan-3-S-exo-carbonsäure- tert.butylester werden in 15 ml wasserfreiem Ethanol gelöst. Man stellt die Lösung mit ethanolischem Kaliumhydroxid auf pH 7,0 und gibt 0,7 g pulverisiertes Molekularsieb (4 Å) und anschließend 5 mmol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25°C wird die Lösung filtriert und das Lösungsmittel entfernt. Der Rückstand wird in Essigester/Wasser aufgenommen. Nach dem Eindampfen der Essigesterphasen wird der Rückstand an Kieselgel mit Essigester/Cyclohexan 1:4 chromatographiert.

Die $^1$H-NMR-Daten stimmen mit den Daten der Verbindung aus Beispiel 13 überein.

Beispiel 23

N-(1-S-Carbethoxy-3-phenyl-3-oxo-propyl)-S-alanyl-2-azabicyclo [2.2.1] heptan-3-S-endo-carbonsäure-tert.butylester

10 mmol Acetophenon, 10 mmol Glyoxylsäureethylester und 10 mmol S-Alanyl-2-azabicyclo [2.2.1]heptan-3-S-carbonsäure tert.-butylester werden in 30 ml Eisessig 36 Stunden auf 45°C erhitzt. Nach dem Einengen im Vakuum wird mit Natriumbicarbonatlösung neutral gestellt und mit Essigester extrahiert. Die Essigesterphase wird eingeengt und an Kieselgel mit Essigester/Cyclohexan 1:1 als Elutionsmittel chromatographiert.

Die NMR-Daten stimmen mit den Daten der Verbindung aus Beispiel 17 überein.

Beispiel 24

N-(1-S-Carbethoxy-3-R,S-hydroxy-3-phenyl-propyl)-S-alanyl-2-azabicyclo [2.2.1] heptan-3-S-exo-carbonsäure

0,5 g N-(1-S-Carbethoxy-3-oxo-3-phenyl-propyl)-S-alanyl-2-azabicyclo [2.2.1] heptan-3-S-exo-carbonsäure werden in 5 ml wäßrigem Ethanol gelöst und 0,1 g Natriumborhydrid zugegeben. Es wird 14 Stunden bei Raumtemperatur gerührt. Danach wird mit Essigester versetzt, die Essigesterlösung mit Wasser gewaschen, getrocknet und eingedampft. Das Rohprodukt wird über Kieselgel mit Essigester/Methanol 9:1 als Elutionsmittel filtriert.
Ausbeute: 0,3 g.

Beispiel 25

2-Aza-bicyclo [2.2.1] heptan-3-carbonsäure

a) 2-(p-Toluolsulfonyl)-2-azabicyclo [2.2.1]hept-5-en-3-carbonsäure-butylester

28 g N- (Butyloxycarbonylmethylen)-p-toluolsulfonamid und 20 ml Cyclopentadien werden in 50 ml Toluol 12,5 Stunden auf 80°C erhitzt. Nach Abkühlen wird eingeengt.

1H-NMR-Daten: 7,4-6,9 (m, 4 H);
5,6-4,2 (m, 4 H);
4,1 (t, J=7Hz, 2 H);
2,3 (s, 3 H);
2,4-1,2 (m, 5 H);
1,0 (t, 3 H).

b) 2-(p-Toluolsulfonyl)-2-azabicyclo [2.2.1]heptan-3-carbonsäure-butylester

15 g der Verbindung aus Beispiel 25 a werden in 200 ml Essigester mit 0,5 g Palladium auf Kohle (10 %) bei Raumtemperatur und Normaldruck hydriert. Nach Abfiltrieren des Katalysators wird eingeengt.

$^1$H-NMR-Daten: 7,4-6,9 (m, 4 H);
5,3-4,2 (m, 2 H);
4,1 (t, 3 H);
3,9-3,3 (m, 1 H);
2,3 (s, 3 H);
2,4-1,2 (m, 9 H);
1,0 (t, 3 H).

c) 2-Azabicyclo [2.2.1]heptan-3-carbonsäure

4 g der Verbindung aus Beispiel 25 b werden mit 1,2

Äquivalenten KOH in 20 ml Wasser 4 Stunden auf 60°C erwärmt. Nach Neutralisieren mit 1N HCl wird eingeengt. Zum Rückstand werden 50 ml flüssiger Ammoniak gegeben und soviel Natrium bis die blaue Farbe bestehen bleibt. Nach 3 Stunden bei -30°C wird wasserfreies Natriumacetat zugesetzt, bis die Farbe verschwindet. Nach Abdampfen des Ammoniaks wird das Rohprodukt auf einer sauren Ionenaustauschersäule gereinigt. Die analytischen Daten stimmen mit der Verbindung aus Beispiel 1 überein.

Beispiel 26

2-Azabicyclo [2.2.1] heptan-3-exo-carbonsäure

a) 2-Azabicyclo [2.2.1] heptan-3-carbonsäure-ethylester

8 g 2-Azabicyclo [2.2.1] hept-5-en-3-carbonsäure-ethylester (Tetrahedron Lett. 1981, 4607) werden in 200 ml Essigester mit 0,5 g Palladium auf Tierkohle als Katalysator bei Raumtemperatur und Normaldruck hydriert. Nach Aufnahme von 2 Mol Wasserstoff wird der Katalysator abfiltriert und das Filtrat eingeengt. Ausbeute: 6,4 g.

$^1$H-NMR-Daten: 4,2 (q, 2 H);
3,8-3,5 (m, 1 H);
2,9-2,6 (m, 1 H);
2,9-1,3 (m, 7 H);
1,2 (t, 3 H).

b) 2-Azabicyclo [2.2.1] heptan-3-exo-carbonsäure

6,4 g der Verbindung aus Beispiel 26a werden analog den in Beispiel 1 f beschriebenen Verfahren hydrolysiert. Die analytischen Daten stimmen mit den der Verbindung aus Beispiel 2 überein.

Nach der in den Beispielen 5 und 7 beschriebenen Verfahrensweise werden weiterhin unter Verwendung der entsprechenden Ausgangsstoffe hergestellt:

Beispiel 27

N-(1-S-Carbethoxy-3-phenyl-propyl)-O-methyl-S-tyrosyl-2-azabicyclo[2.2.1]heptan-3-S-exo-carbonsäure-Hydrochlorid

m/e (trimethylsilyliert): 580 ($M^+$), 340 (100 %).

Beispiel 28

N-(1-S-Carbethoxy-3-phenyl-propyl)-O-ethyl-S-tyrosyl-2-azabicyclo[2.2.1]heptan-3-S-endo-carbonsäure-Hydrochlorid

m/e (trimethylsilyliert): 594 ($M^+$), 354 (100 %).

Beispiel 29

N-(1-S-Carbethoxy-3-cyclohexyl-propyl)-O-methyl-S-tyrosyl-2-azabicyclo[2.2.1]heptan-3-S-exo-carbonsäure-Hydrochlorid

m/e (trimethylsilyliert): 586 ($M^+$), 346 (100 %).

Beispiel 30

N-(1-S-Carbethoxy-3-cyclohexyl-propyl)-O-ethyl-S-tyrosyl-2-azabicyclo[2.2.1]heptan-3-S-endo-carbonsäure-Hydrochlorid

m/e (trimethylsilyliert): 600 ($M^+$), 360 (100 %).

Beispiel 31

N-(1-S-Carbethoxy-3-cyclohexyl-propyl)-S-alanyl-2-azabicyclo[2.2.1]heptan-3-S-exo-carbonsäure-Hydrochlorid

m/e (trimethylsilyliert): 480 ($M^+$), 240 (100 %).

Beispiel 32

N-(1-S-Carbethoxy-3-cyclohexyl-propyl)-2-alanyl-2-azabicyclo[2.2.1]heptan-3-S-endo-carbonsäure-Hydrochlorid

m/e (trimethylsilyliert): 480 ($M^+$), 240 (100 %).

Beispiel 33

N-(1-S-Carbethoxy-3-cyclopentyl-propyl)-2-alanyl-2-azabicyclo[2.2.1]heptan-3-S-exo-carbonsäure-Hydrochlorid

m/e (trimethylsilyliert): 466 ($M^+$), 224 (100 %).

Beispiel 34

N-(1-S-Carbethoxy-3-cyclopentyl-propyl)-2-alanyl-2-azabicyclo[2.2.1]heptan-3-S-endo-carbonsäure-Hydrochlorid

m/e (trimethylsilyliert): 466 ($M^+$), 224 (100 %).

Beispiel 35

N-(1-S-Carbethoxy-3-(2-methyl-phenyl)-propyl)-S-alanyl-2-azabicyclo[2.2.1]heptan-3-S-endo-carbonsäure-Hydrochlorid

m/e (trimethylsilyliert): 488 ($M^+$), 248 (100 %).

Beispiel 36

N-(1-S-Carbethoxy-3-(4-methoxy-phenyl)-propyl)-S-alanyl-2-azabicyclo[2.2.1]heptan-3-S-exo-carbonsäure-Hydrochlorid

m/e (trimethylsilyliert): 504 ($M^+$), 264 (100 %).

Beispiel 37

N-(1-S-Carbethoxy-3-(4-fluor-phenyl-propyl)-S-alanyl-2-azabicyclo/2.2.1/heptan-3-S-endo-carbonsäure-Hydrochlorid

m/e (trimethylsilyliert): 492 ($M^+$), 252 (100 %).

Beispiel 38

N-(1-S-Carbethoxy-3-(3,4-dimethoxyphenyl-propyl)-S-alanyl-2-azabicyclo/2.2.1/heptan-3-S-exo-carbonsäure-Hydrochlorid

m/e (trimethylsilyliert): 534 ($M^+$), 294 (100 %).

Beispiel 39

N-(1-S-Carbethoxy-3-(2,6-dichlorphenyl-propyl)-S-alanyl-2-azabicyclo/2.2.1/heptan-3-S-exo-carbonsäure-Hydrochlorid

m/e (trimethylsilyliert): 542 ($M^+$), 272 (100 %).

Beispiel 40

N-(1-S-Carbethoxy-3-(3,4-methylendioxyphenyl-propyl)-S-alanyl-azabicyclo/2.2.1/heptan-3-S-endo-carbonsäure-Hydrochlorid

m/e (trimethylsilyliert): 516 ($M^+$), 278 (100 %).

Beispiel 41

N-(1-S-Carbethoxy-3-phenyl-propyl)-S-lysyl-bicyclo/2.2.1/-heptan-3-S-exo-carbonsäure-Hydrochlorid

m/e (trimethylsilyliert): 537 ($M^+$), 292 (100 %).

Beispiel 42

N-(1-S-Carboxy-3-phenyl-propyl)-S-lysyl-bicyclo/2.2.1/-heptan-3-S-exo-carbonsäure

m/e (trimethylsilyliert): 581 ($M^+$), 336 (100 %).

Beispiel 43

N-(1-S-Carbethoxy-3-phenyl-propyl)-S-lysyl-bicyclo/2.2.1/-heptan-3-S-endo-carbonsäure-Hydrochlorid

m/e (trimethylsilyliert): 537 ($M^+$), 292 (100 %).

Beispiel 44

N-(1-S-Carboxy-3-phenyl-propyl)-S-lysyl-bicyclo/2.2.1/-heptan-3-S-endo-carbonsäure-Hydrochlorid

m/e (trimethylsilyliert): 581 ($M^+$), 336 (100 %).

Beispiel 45

N-(1-S-Carbethoxy-3-cyclohexyl-propyl)-S-lysyl-bicyclo-/2.2.1/-heptan-3-S-exo-carbonsäure-Hydrochlorid

m/e (trimethylsilyliert): 543 ($M^+$), 298 (100 %).

Beispiel 46

N-(1-S-Carboxy-3-cyclohexyl-propyl)-S-lysyl-bicyclo-/2.2.1/-heptan-3-S-exo-carbonsäure-Hydrochlorid

m/e (trimethylsilyliert): 597 ($M^+$), 343 (100 %).

Beispiel 47

N-(1-S-Carbethoxy-3-cyclohexyl-propyl)-S-lysyl-bicyclo-/2.2.1/-heptan-3-S-endo-carbonsäure-Hydrochlorid

m/e (trimethylsilyliert): 543 ($M^+$), 298 (100 %).

Beispiel 48

N-(1-S-Carboxy-3-cyclohexyl-propyl)-S-lysyl-bicyclo-/2.2.1/-heptan-3-S-endo-carbonsäure

m/e (trimethylsilyliert): 587 ($M^+$), 343 (100 %).

PATENTANSPRÜCHE:

1. Verbindung der Formel I

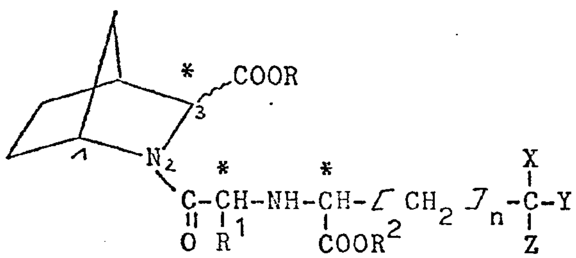

(I)

in welcher

n = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)- Cycloalkenyl, ($C_5$ bis $C_7$)-Cycloalkyl-($C_1$ bis $C_4$)-alkyl, ($C_6$-$C_{12}$)-Aryl oder teilhydriertes ($C_6$-$C_{12}$)-Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden $\alpha$-Aminosäure,

$R^2$ = Wasserstoff, ($C_1$ bis $C_6$)-Alkyl, ($C_2$ bis $C_6$)-Alkenyl oder ($C_6$-$C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$-$C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkyl-amino und/oder Methylendioxy mono-, di- oder trisubstituiert sein kann, oder 3-Indolyl bedeuten, sowie deren physiologisch unbedenkliche Salze.

2. Verbindung der Formel I gemäß Anspruch 1, dadurch gekennzeichnet, daß das C-Atom in Position 3 des bicyclischen Ringsystems sowie die mit einem Stern markierten C-Atome der Seitenkette jeweils S-Konfiguration aufweisen.

3. Verbindung der Formel I gemäß einem der Ansprüche 1 und 2, dadurch gekennzeichnet, daß

n = 1

R = Wasserstoff oder ($C_1$ bis $C_4$)-Alkyl,

$R_1$ = Wasserstoff, ($C_1$ bis $C_3$)-Alkyl, die gegebenenfalls acylierte Seitenkette von Lysin, ($C_2$ oder $C_3$)-Alkenyl, Benzyl, die O-($C_1$-$C_6$)-alkylierte Seitenkette von Tyrosin, 4-Methoxybenzyl, 4-Ethoxybenzyl, Phenethyl, 4-Amino-butyl oder Benzoylmethyl

$R_2$ = Wasserstoff, ($C_1$ bis $C_4$)-Alkyl oder Benzyl,

X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$) alkyl-amino, Nitro und/oder Methylendioxy mono- oder disubstituiert oder im Falle von Methoxy, trisubstituiert sein kann, bedeuten.

4. Verbindung der Formel I gemäß Anspruch 1, dadurch gekennzeichnet, daß

n = 1,

R = Wasserstoff,

$R^1$ = Methyl, 4-Methoxybenzyl oder 4-Ethoxybenzyl und
$R^2$ = Wasserstoff oder Ethyl bedeuten und die chiralen, mit einem Stern (*) markierten C-Atomen die S-Konfiguration besitzen.

5. Verfahren zur Herstellung der Verbindungen der Formel I gemäß einem der Ansprüche 1 bis 4, dadurch gekennzeichnet,
a) daß man eine Verbindung der Formel II,

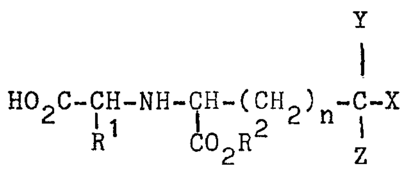

(II)

worin n, $R^1$, $R^2$, X, Y und Z die Bedeutung wie in Formel I haben mit einer Verbindung der Formel III,

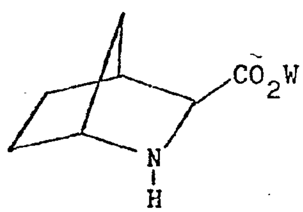

(III)

in welcher
W = Wasserstoff oder einen sauer oder hydrogenolytisch abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV

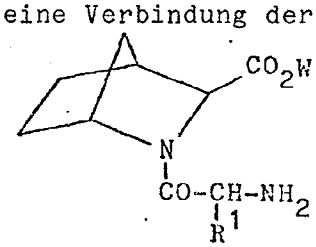

(IV)

in welcher $R^1$ die Bedeutung wie in Formel I und W die Bedeutung wie in Formel III besitzen, mit einer Verbindung der Formel V,

$$R^2O_2C-CH=CH-CO-X \qquad (V)$$

worin $R^2$ und X die Bedeutungen wie in Formel I haben, umgesetzt und anschließend gegebenenfalls W und/oder $R^2$ unter Bildung der freien Carboxygruppen abgespaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC-CO_2R^2 \quad (VI) \qquad X-CO-CH_3 \quad (VII)$$

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

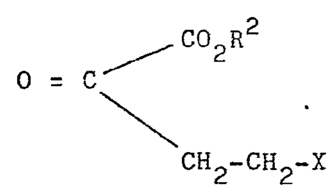

(VIII)

worin $R^2$ und X die Bedeutungen wie in Formel I besitzen. umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert;

und die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt.

6. Verwendung einer Verbindung gemäß einem der Ansprüche 1 bis 4 als Heilmittel.

7. Verbindung gemäß einem der Ansprüche 1 bis 4 zur Verwendung als Heilmittel.

8. Mittel, enthaltend eine Verbindung gemäß einem der Ansprüche 1 bis 4.

9. Mittel, enthaltend eine Verbindung gemäß einem der Ansprüche 1 bis 4 in Kombination mit einem Diuretikum.

10. Verwendung einer Verbindung gemäß einem der Ansprüche 1 bis 4, in Kombination mit einem Diuretikum als Heilmittel.

11. Verbindungen der Formeln IIIa und IIIb

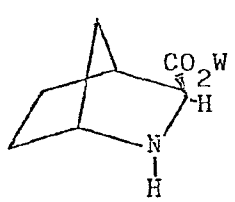

(IIIa)

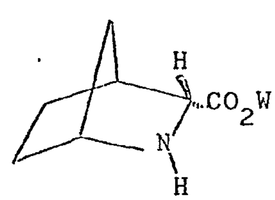

(IIIb)

in welchen W Wasserstoff oder einen sauer oder hydrogenolytisch abspaltbaren Rest bedeutet.

12. Verfahren zur Herstellung von Verbindungen der Formeln IIIa und IIIb gemäß Anspruch 11, dadurch gekennzeichnet, daß man

a) eine Verbindung der Formeln IXa oder IXb

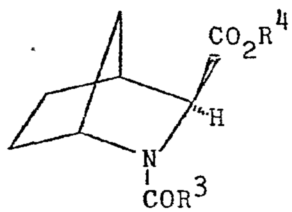

(IXa)

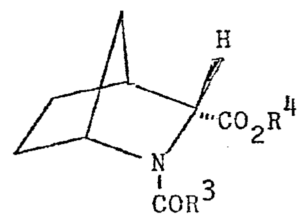

(IXb)

in welchen $R^3$ ($C_1$ bis $C_6$)-Alkyl-, Phenyl- oder Trifluoracetyl- und $R^4$ einen ($C_1$ bis $C_6$)-Alkyl- oder Benzylrest bedeuten mit einer Mineralsäure oder mit einer starken Base verseift und die erhaltenen Aminosäuren der Formeln IIIa bzw. IIIb, in welchen W Wasserstoff bedeutet gegebenfalls in an sich bekannter Weise verestert oder ein Gemisch von Verbindungen der Formeln IXa und IXb verseift und die Aminosäuren (IIIa und IIIb/ W = Wasserstoff) trennt und anschließend gegebenenfalls verestert oder das erhaltene Aminosäurengemisch zunächst wie oben verestert und die Ester der Formeln IIIa und IIIb (W ≠ Wasserstoff) auftrennt oder

b) eine Verbindung der Formel X

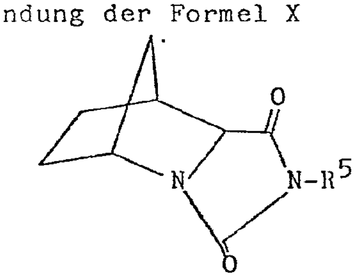

(X)

in welcher $R^5$ einen ($C_1$ bis $C_6$)-Alkyl-, Aryl-($C_1$ bis $C_3$)-alkyl-, Phenyl-, 4-Methoxyphenyl- oder 4-Chlorphenylrest darstellt, mit einem Alkali- oder Erdalkalihydroxid in Wasser oder Gemischen davon mit einem organischen Lösungsmittel verseift, und die erhaltene Aminosäure (IIIa oder b; W = Wasserstoff) gegebenenfalls nach üblichen Methoden der Aminosäurechemie verestert oder

c) eine Verbindung der Formeln XIa bzw. XIb

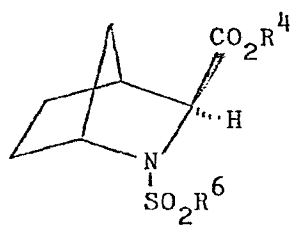

(XIa)

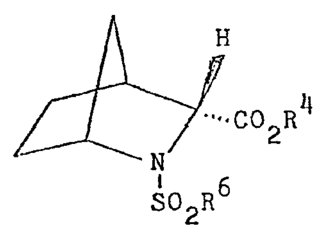

(XIb)

in welcher $R^4$ die gleiche Bedeutung wie in Formeln IXa und IXb besitzt und $R^6$ einen Phenyl-, 4-Methylphenyl- oder 4-Chlorphenylrest darstellt, zunächst mit einem Alkali- oder Erdalkalimetall, dann mit einem Alkali- oder Erdalkalihydroxid umsetzt, oder die Reihenfolge der beschriebenen Reaktionsschritte umkehrt und gegebenenfalls wie oben verestert, oder

d) eine Verbindung der Formel XIIa oder XIIb

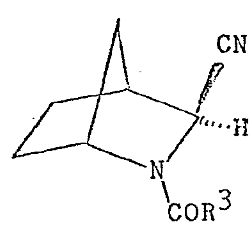

(XIIa)

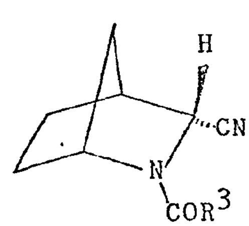

(XIIb)

mit einer Mineralsäure oder mit einer starken Base verseift und die erhaltenen Aminosäuren (IIIa bzw. IIIb/W = Wasserstoff) gegebenenfalls wie oben verestert oder ein Gemisch von Verbindungen der Formeln XIIa und XIIb unter den angegebenen Reaktionsbedingungen verseift und die Aminosäuren (IIIa und IIIb/W = Wasserstoff) trennt oder das erhaltene Aminosäurengemisch zunächst wie oben verestert und die erhaltenen Ester (IIIa und IIIb/W ≠ Wasserstoff) auftrennt.

PATENTANSPRÜCHE für Österreich:

1. Verfahren zur Herstellung von Verbindungen der Formel I

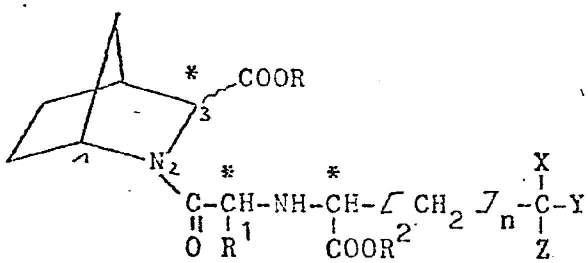

(I)

in welcher

n = 0 oder 1,

R = Wasserstoff, ($C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_5$ bis $C_9$)-Cycloalkyl, ($C_5$ bis $C_9$)- Cycloalkenyl, ($C_5$ bis $C_7$)-Cycloalkyl-($C_1$ bis $C_4$)-alkyl, ($C_6$-$C_{12}$)-Aryl oder teilhydriertes ($C_6$-$C_{12}$)-Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine gegebenenfalls geschützte Seitenkette einer natürlich vorkommenden α-Aminosäure,

$R^2$ = Wasserstoff, ($C_1$ bis $C_6$)-Alkyl, ($C_2$ bis $C_6$)-Alkenyl oder ($C_6$-$C_{12}$)-Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$-$C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)-alkyl-amino und/oder Methylendioxy mono-, di- oder trisubstituiert sein kann, oder 3-Indolyl bedeuten, sowie deren physiologisch unbedenklichen Salzen, dadurch gekennzeichnet,

a) daß man eine Verbindung der Formel II,

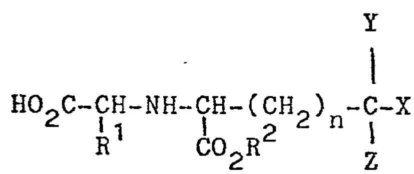

(II)

worin n, $R^1$, $R^2$, X, Y und Z die Bedeutung wie in Formel I haben mit einer Verbindung der Formel III,

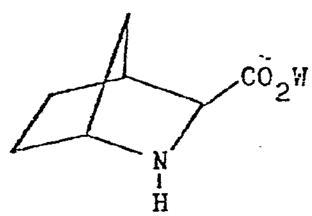

(III)

in welcher

W = Wasserstoff oder einen sauer oder hydrogenolytisch abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV

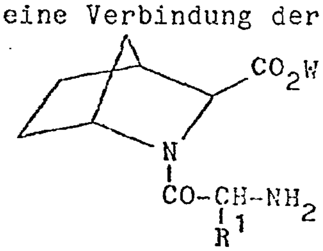

(IV)

in welcher $R^1$ die Bedeutung wie in Formel I und W die Bedeutung wie in Formel III besitzen, mit einer Verbindung der Formel V,

$$R^2O_2C-CH=CH-CO-X \quad (V)$$

worin $R^2$ und X die Bedeutungen wie in Formel I haben, umgesetzt und anschließend gegebenenfalls W und/oder $R^2$ unter Bildung der freien Carboxygruppen abgespaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$$OHC-CO_2R^2 \quad (VI) \qquad X-CO-CH_3 \quad (VII)$$

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

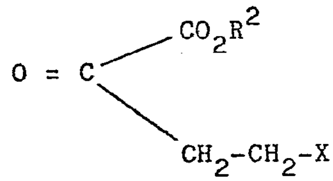

(VIII)

worin $R^2$ und X die Bedeutungen wie in Formel I besitzen. umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert;

und die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt.

2. Verfahren gemäß Anspruch 1, dadurch gekennzeichnet, daß Verbindungen der Formel I hergestellt werden, in welcher das C-Atom in Position 3 des bicyclischen Ringsystems sowie die mit einem Stern markierten C-Atome der Seitenkette jeweils S-Konfiguration aufweisen.

3. Verfahren gemäß Anspruch 1 oder 2, dadurch gekennzeichnet, daß Verbindungen der Formel I hergestellt werden, in welcher

n = 1

R = Wasserstoff oder ($C_1$ bis $C_4$)-Alkyl,

$R^1$ = Wasserstoff, ($C_1$ bis $C_3$)-Alkyl, die gegebenenfalls acylierte Seitenkette von Lysin, ($C_2$ oder $C_3$)-Alkenyl, Benzyl, die O-($C_1$-$C_6$)-alkylierte Seitenkette von Tyrosin, 4-Methoxybenzyl, 4-Ethoxybenzyl, Phenethyl, 4-Amino-butyl oder Benzoylmethyl

$R^2$ = Wasserstoff, ($C_1$ bis $C_4$)-Alkyl oder Benzyl,
X = Cyclohexyl oder Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$) alkyl-amino, Nitro und/oder Methylendioxy mono- oder disubstituiert oder im Falle von Methoxy, trisubstituiert sein kann, bedeuten.

4. Verfahren gemäß Anspruch 1, dadurch gekennzeichnet, daß Verbindungen der Formel I hergestellt werden, in welcher
n = 1,
R = Wasserstoff,
$R^1$ = Methyl, 4-Methoxybenzyl oder 4-Ethoxybenzyl und
$R^2$ = Wasserstoff oder Ethyl bedeuten und die chiralen, mit einem Stern (*) markierten C-Atomen die S-Konfiguration besitzen.

5. Verfahren gemäß einem der Ansprüche 1 bis 4 zur Herstellung einer Verbindung der Formel I zur Verwendung als Heilmittel.

6. Verfahren zur Herstellung eines Mittels, enthaltend eine Verbindung der Formel I, dadurch gekennzeichnet, daß man diese in eine geeignete Darreichungsform bringt.

7. Verfahren gemäß Anspruch 6, dadurch gekennzeichnet, daß man eine Verbindung der Formel I in Kombination mit einem Diuretikum in eine geeignete Darreichungsform bringt.

8. Verfahren zur Herstellung von Verbindungen der Formeln IIIa und IIIb

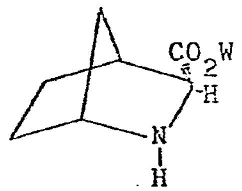

(IIIa)

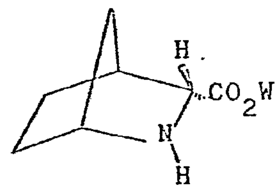

(IIIb)

in welchen W Wasserstoff oder einen sauer oder hydrogenolytisch abspaltbaren Rest bedeutet, dadurch gekennzeichnet, daß man

a) eine Verbindung der Formeln IXa oder IXb

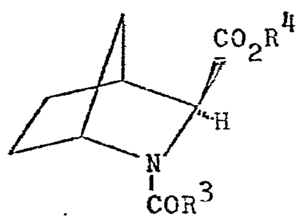

(IXa)

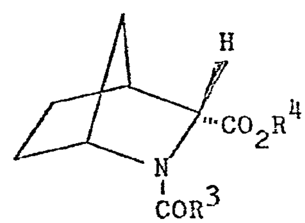

(IXb)

in welchen $R^3$ ($C_1$ bis $C_6$)-Alkyl-, Phenyl- oder Trifluoracetyl- und $R^4$ einen ($C_1$ bis $C_6$)-Alkyl- oder Benzylrest bedeuten mit einer Mineralsäure oder mit einer starken Base verseift und die erhaltenen Aminosäuren der Formeln IIIa bzw. IIIb, in welchen W Wasserstoff bedeutet gegebenfalls in an sich bekannter Weise verestert oder ein Gemisch von Verbindungen der Formeln IXa und IXb verseift und die Aminosäuren (IIIa und IIIb/ W = Wasserstoff) trennt und anschließend gegebenenfalls verestert oder das erhaltene Aminosäurengemisch zunächst wie oben verestert und die Ester der Formeln IIIa und IIIb (W ≠ Wasserstoff) auftrennt oder

b) eine Verbindung der Formel X

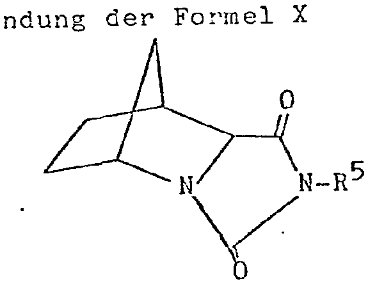

(X)

in welcher $R^5$ einen ($C_1$ bis $C_6$)-Alkyl-, Aryl-($C_1$ bis $C_3$)-alkyl-, Phenyl-, 4-Methoxyphenyl- oder 4-Chlorphenylrest darstellt, mit einem Alkali- oder Erdalkalihydroxid in Wasser oder Gemischen davon mit einem organischen Lösungsmittel verseift, und die erhaltene Aminosäure (IIIa oder b; W = Wasserstoff) gegebenenfalls nach üblichen Methoden der Aminosäurechemie verestert oder

c) eine Verbindung der Formeln XIa bzw. XIb

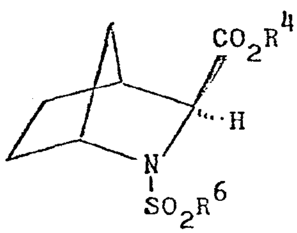

(XIa)

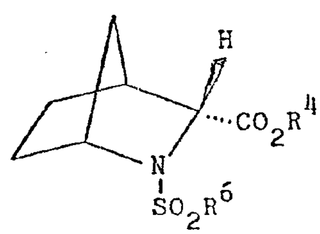

(XIb)

in welcher $R^4$ die gleiche Bedeutung wie in Formeln IXa und IXb besitzt und $R^6$ einen Phenyl-, 4-Methylphenyl- oder 4-Chlorphenylrest darstellt, zunächst mit einem Alkali- oder Erdalkalimetall, dann mit einem Alkali- oder Erdalkalihydroxid umsetzt oder die Reihenfolge der beschriebenen Reaktionsschritte umkehrt und gegebenenfalls wie oben verestert oder

d) eine Verbindung der Formel XIIa oder XIIb

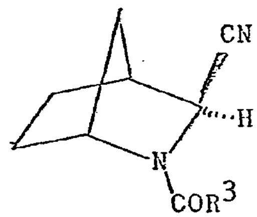

(XIIa)

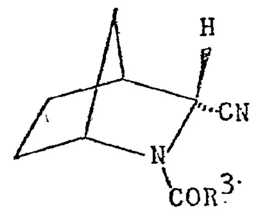

(XIIb)

mit einer Mineralsäure oder mit einer starken Base verseift und die erhaltenen Aminosäuren (IIIa bzw. IIIb/W = Wasserstoff) gegebenenfalls wie oben verestert oder ein Gemisch von Verbindungen der Formeln XIIa und XIIb unter den angegebenen Reaktionsbedingungen verseift und die Aminosäuren (IIIa und IIIb/W = Wasserstoff) trennt oder das erhaltene Aminosäurengemisch zunächst wie oben verestert und die erhaltenen Ester (IIIa und IIIb/W ≠ Wasserstoff) auftrennt.